# EUROPEAN PATENT APPLICATION

(11) **EP 2 299 530 A1**
(43) Date of publication of application: **23.03.2011**
(21) Application number: 09742673.8
(22) Date of filing: 23.04.2009
(51) Int. Cl.: H01M 8/16, C12N 9/02, C12N 15/00, H01M 4/90

(54) **NOVEL ENZYME ELECTRODE, AND FUEL CELL COMPRISING THE ENZYME ELECTRODE**

(30) Priority: 08.05.2008 JP 2008122574
(71) Applicant: Sony Corporation, Tokyo 108-0075 (JP)
(72) Inventor: NAKAGAWA, Takaaki, Tokyo 108-0075 (JP); KUMITA, Hideyuki, Tokyo 108-0075 (JP); GOTO, Yoshio, Tokyo 108-0075 (JP); SAKAI, Hideki, Tokyo 108-0075 (JP); KAKUTA, Masaya, Tokyo 108-0075 (JP)
(74) Representative: Müller - Hoffmann & Partner
(86) International application number: PCT/JP2009/058065
(87) International publication number: WO 2009/136548

(57) **Abstract**

Provided is an enzyme electrode in which oxidation-reduction reactions proceed with an enzyme acting as a catalyst, and the enzyme is modified to increase affinity and/or reaction rate with a reaction substrate or an electron transfer mediator by adding or inserting at least one codon encoding a particular amino acid residue to or into a base sequence encoding the enzyme, and is immobilized. Since the oxidation-reduction reactions on the electrode proceed highly efficiently, the enzyme electrode can cause to increase the obtained output of electric energy and thus can be suitably used in all types of fuel cells, biosensors, and electronic apparatuses.

SEQUENCE LISTING

<110>  SONY Corporation
       Takaaki, NAKAGAWA
       Hideyuki, KUMITA
       Yoshio, GOTO
       Hideki, SAKAI
       Masaya, KAKUTA

<120>  ENZYME ELECTRODE, AND FUEL CELL

<130>  07904977

<160>  4

<170>  PatentIn version 3.1

<210>  1
<211>  44
<212>  DNA
<213>  Artificial Sequence

<220>
<223>  vector for Lys4-BOD

<400>  1
gggactagtc acttttcttt tttctcgtca gctgcggcgt aagg                        44


<210>  2
<211>  50
<212>  DNA
<213>  Artificial Sequence

<220>
<223>  vector for Lys6-BOD

<400>  2
   gggactagtc acttttcttt tttctttttc tcgtcagctg cggcgtaagg                   50


<210>  3
<211>  56
<212>  DNA
<213>  Artificial Sequence

<220>
<223>  vecter for Lys8-BOD

<400>  3
   gggactagtc acttttcttt tttctttttc tttttctcgt cagctgcggc gtaagg            56


<210>  4
<211>  62
<212>  DNA
<213>  Artificial Sequence

<220>
<223>  vecter for Lys10-BOD

<400> 4
gggactagtc actttttctt tttctttttc tttttctttt tctcgtcagc tgcggcgtaa 60

gg 62

SEQUENCE LISTING

<110> SONY Corporation
Takaaki. NAKAGAWA
Hideyuki. KUMITA
Yoshio. GOTO
Hideki. SAKAI
Masaya. KAKUTA

<120> ENZYME ELECTRODE. AND FUEL CELL

<130> 07904977

<160> 4

<170> PatentIn version 3.1

<210> 1
<211> 44
<212> DNA
<213> Artificial Sequence

<220>
<223> vector for Lys4-BOD

<400> 1
gggactagtc actttttctt tttctcgtca gctgcggcgt aagg 44

<210> 2
<211> 50
<212> DNA
<213> Artificial Sequence

<220>
<223> vector for Lys6-BOD

<400> 2
gggactagtc actttttctt tttctttttc tcgtcagctg cggcgtaagg 50

<210> 3
<211> 56
<212> DNA
<213> Artificial Sequence

<220>
<223> vecter for Lys8-BOD

<400> 3
gggactagtc actttttctt tttctttttc tttttctcgt cagctgcggc gtaagg 56

<210> 4
<211> 62
<212> DNA
<213> Artificial Sequence

<220>
<223> vecter for Lys10-BOD

<400> 4
gggactagtc actttttctt tttctttttc tttttctttt tctcgtcagc tgcggcgtaa 60

gg 62

## Description

### Technical Field

The present invention relates to enzyme electrodes. More specifically, it relates to an enzyme electrode in which oxidation-reduction reactions proceed with an enzyme acting as a catalyst and which is improved to achieve higher output, and a fuel cell that uses the enzyme electrode so that it can achieve higher output.

### Background Art

In recent years, fuel cells (hereinafter referred to as "biofuel cells") that have an oxidoreductase immobilized on at least one of a negative electrode and a positive electrode to serve as a catalyst have drawn much attention as next-generation fuel cells that have high capacities and safety since electrons can be efficiently taken out from fuels, such as glucose and ethanol, which do not readily react to regular industrial catalysts.

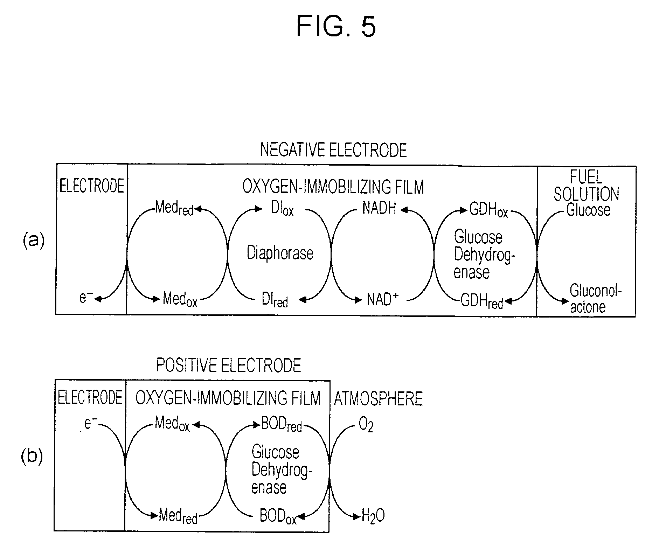

Using Fig. 5, a reaction scheme of a typical biofuel cell is described. In the biofuel cell that uses glucose as a fuel shown in Fig. 5, oxidation reaction of glucose proceeds at the negative electrode (Fig. 5(a)) and reduction reaction of oxygen (O₂) in atmosphere proceeds at the positive electrode (Fig. 5(b)).

The flow of electrons are described in detail as follows. At the negative electrode, electrons are successively transferred to glucose, a glucose dehydrogenase, nicotinamide adenine dinucleotide (NAD+), diaphorase, an electron transfer mediator, and then to an electrode (carbon) in that order.

In contrast, at the positive electrode, electrons released from the negative electrode are successively transferred to the electrode (carbon), the electron transfer mediator, and then to bilirubin oxidase (BOD) in that order and reduction reaction proceeds using the electrons and oxygen supplied from outside to generate electric energy.

While such biofuel cells are drawing attentions as highly safe fuel cells, they have a problem that the output is low compared to other fuel cells. Thus, recently, studies are being made to make biofuel cells that have high output (e.g., refer to PTL 1 and PTL 2).

For example, according to a biofuel cell described in PTL 1, an electrode is constituted by a conductive member (a metal, a conductive polymer, a metal oxide, a carbon material, or the like) having a porous structure, and an enzyme, an electron transfer mediator, etc., are immobilized in pores thereof to increase the enzyme-carrying density per effective area and improve the current density.

According to a biofuel cell described in PTL 2, a cathode electrode is constituted by a porous material such as carbon and an enzyme and an electron transfer mediator immobilized thereon, and at least part of the cathode electrode is arranged to contact air or oxygen that serves as a reaction substrate in a gas phase so that high electrode characteristics can be sufficiently exhibited.

Aside from the technology that achieves higher output by modifying the structure of the electrode, there is a technology that causes biofuel cells to achieve higher output by modifying the enzyme itself to be immobilized on the electrode. For example, PTL 3 discloses preparation of a protein through fusion of a dehydrogenase and diaphorase and that the fusion protein is immobilized on a negative electrode to efficiently carry out the electron transfer reaction from the substrate to the electrode and to thereby cause biofuel cells to have higher output.

Furthermore, PTL 4 discloses a technology that enables biofuel cells to have higher outputs by immobilizing, on an electrode, a binding protein constituted by binding two or more different types of enzyme proteins so as to efficiently carry out the electron transfer reaction from the substrate to the electrode due to a small relative distance between the two types of enzyme proteins forming the binding protein.

It should be noted that in order to increase the output of the biofuel cells, the reaction efficiency between the enzyme to be immobilized on the electrode and the reaction substrate (in particular, the electron transfer mediator) must be improved. The reaction between the enzyme and the reaction substrate is expressed by affinity (Km) and reaction rate (kcat) and these two parameters are determined by the combination of the enzyme and the reaction substrate; therefore, the enzyme and the reaction substrate (in particular, the electron transfer mediator) that are compatible with each other must be searched.

However, in order to increase the output of the biofuel cells, there is a necessity to obtain high current while retaining the potential determined by the electron transfer mediator or the like at a high level; thus, under current circumstances, the combination of the enzyme and the reaction substrate is limited and there is a limit as to the output value.

### Citation List

### Patent Literature

PTL 1: Japanese Unexamined Patent Application Publication No. 2006-234788
PTL 2: Japanese Unexamined Patent Application Publication No. 2006-93090
PTL 3: Japanese Unexamined Patent Application Publication No. 2007-163185
PTL 4: Japanese Unexamined Patent Application Publication No. 2007-163268

### Summary of Invention

### Technical Problem

As mentioned above, the technology for increasing output of biofuel cells has been developed from various angles but the fundamental principle of the biofuel cells for obtaining electric energy is based on the oxidation-reduction reactions on the electrodes; thus, a basic solution cannot be obtained without increasing the reaction efficiency between the reaction substrate and the enzyme on the electrode.

Thus, a main object of the present invention is to artificially increase the compatibility between the reaction substrate and the enzyme to be immobilized on an electrode of a biofuel cell so as to increase the reaction efficiency on the electrode and thereby enable the biofuel cell to achieve higher outputs.

### Technical Solution

### Solution to Problem

The inventors of the present application have conducted extensive researches for increasing the reaction efficiency between the reaction substrate and the enzyme on the electrode to achieve the aforementioned object, and, as a result, succeeded in artificially increasing the compatibility between the enzyme and the reaction substrate by focusing on the electrostatic interactions, hydrophilic and hydrophobic interactions, etc., between the enzyme and the reaction substrate and made the present invention.

First, the present invention provides an enzyme electrode in which oxidation-reduction reactions proceed with an enzyme acting as a catalyst and in which the enzyme is modified to increase affinity and/or reaction rate with a reaction substrate or an electron transfer mediator by adding or inserting at least one codon encoding a particular amino acid residue to or into a base sequence encoding the enzyme, and is immobilized.
The method for modifying the enzyme is not particularly limited as long as it is a method that increases the affinity and/or reaction rate with the reaction substrate or the electron transfer mediator by adding or inserting at least one codon encoding a particular amino acid residue into a base sequence encoding the enzyme; however, for example, electrostatic interactions can be utilized to increase the affinity and/or reaction rate with the reaction substrate or the electron transfer mediator.
The amino acid residue is not particularly limited; however, for example, an amino acid residue that has a charge opposite to that of the reaction substrate or the electron transfer mediator can be used.
The specific types of the electron transfer mediator and the amino acid residue are also not particularly limited; however, for example, when a mediator whose oxidized form or reduced form is an anion is used as the electron transfer mediator, the amino acid residue can be selected from a lysine residue, a histidine residue, and an arginine residue.
Furthermore, a method for increasing the affinity and/or reaction rate with the reaction substrate or the electron transfer mediator by utilizing hydrophilic or hydrophobic interactions can be used as the method for modifying the enzyme.
In such a case, when the reaction substrate or the electron transfer mediator is a hydrophilic substance, a hydrophilic amino acid is used as the amino acid residue and when the reaction substrate or the electron transfer mediator is a hydrophobic substance, a hydrophobic amino acid is used as the amino acid residue to increase the affinity and/or reaction rate.

Next, the present invention provides a fuel cell in which oxidation-reduction reactions proceed on an electrode with an enzyme acting as a catalyst and in which the enzyme is modified to increase affinity and/or reaction rate with a reaction substrate or an electron transfer mediator by adding or inserting at least one codon encoding a particular amino acid residue to or into a base sequence encoding the enzyme, and is immobilized on at least one of a positive electrode and a negative electrode.

The technical terms used in the present invention will now be described.

"Modification of enzyme" used in the present invention means that one or several codons that encode amino acid residues are added or inserted so as to change properties such as stability and affinity and/or reaction rate with a reaction substrate or an electron transfer mediator without changing the function of the enzyme protein.

### Advantageous Effects of Invention

An enzyme electrode according to the present invention causes the oxidation-reduction reactions on the electrode to proceed highly efficiently and thus the resulting output of the electrical energy can be increased.

### Brief Description of Drawings

[Fig. 1] Fig. 1 is a graph substituting a drawing and showing the results of DNA sequencing of a Lys6 gene in Example 1.
[Fig. 2] Fig. 2 is a graph substituting a drawing and showing the results of DNA sequencing of a Lys8 gene in Example 1.
[Fig. 3] Fig. 3 is a graph substituting a drawing and showing the results of DNA sequencing of a Lys10 gene in Example 1.
[Fig. 4] Fig. 4 is a photograph substituting a drawing and showing the presence or absence of BOD activities of enzymes expressed from a Lys6-BOD gene, a Lys8-BOD gene, and a Lys10-BOD gene in Example 2.
[Fig. 5] Fig. 5 is a schematic diagram showing a reaction scheme of a typical biofuel cell.

### Description of Embodiments

### <Enzyme electrode>

An enzyme electrode according to the present invention is an electrode in which oxidation-reduction reactions proceed with an enzyme acting as a catalyst, and is an electrode in which an enzyme which has been modified to increase the affinity and/or reaction rate with a reaction substrate or an electron transfer mediator is immobilized.

Modification of the enzyme is done by adding or inserting at least one codon encoding a particular amino acid residue to or into a base sequence encoding the enzyme. In this case, "modification" refers to increasing the affinity and/or reaction rate with the reaction substrate or the electron transfer mediator by adding or inserting at least one particular amino acid residue without changing the properties of the enzyme.

The specific method of the method for modifying the enzyme is not particularly limited as long as it is a method for increasing the affinity and/or reaction rate with the reaction substrate or the electron transfer mediator by adding or inserting at least one codon encoding a particular amino acid residue to or into a base sequence encoding the enzyme; however, for example, the affinity and/or reaction rate with the reaction substrate or the electron transfer mediator can be increased by utilizing the electrostatic interactions or hydrophilic or hydrophobic interactions.

As for a specific method for increasing the affinity and/or reaction rate by utilizing electrostatic interactions, for example, a codon encoding an amino acid residue having a charge opposite to that of the reaction substrate or the electron transfer mediator used can be added to or inserted into a base sequence encoding the enzyme so as to increase affinity and/or reaction rate between the enzyme to be immobilized and the reaction substrate or the electron transfer mediator.

To be more specific, in the case where a reaction substrate or an electron transfer mediator having a negative charge is used, a codon that encodes an amino acid residue having a positive charge is added to or inserted into a base sequence coding the enzyme and in the case where a reaction substrate or an electron transfer mediator having a positive charge is used, a codon that encodes an amino acid residue having a negative charge is added to or inserted into; thus, the affinity and/or reaction rate between the enzyme expressed and the reaction substrate or the electron transfer mediator can be increased.

In addition, since a codon encoding the amino acid residue having a charge is added to or inserted into a base sequence encoding the enzyme, the enzyme can be immobilized on the electrode due to electrostatic interactions and thus the enzyme electrode can be stabilized.

Furthermore, the material needed to immobilize the enzyme on the electrode can be reduced, and, as a result, the cost of the manufacturing process for the enzyme electrode can be reduced.

The electron transfer mediator that can be used in utilizing the electrostatic interactions is not particularly limited. Examples of an electron transfer mediator having a negative charge (mediator whose oxidized form or reduced form is an anion) include pigments such as ABTS (2,2'-azinobis(3-ethylbenzoline-6-sulfonate)) and cyano metal complexes such as hexacyanoferric ions and octacyanotungstate ions and examples of an electron transfer mediator (mediator whose oxidized form or reduced form is a cation) include metal complexes to which pyridine, bipyridine, or the like is coordinated such as [Os (trpy3)]^{3+/2+}, [Os (py)₂(bpy)₂]^{3+/2+}, and [Fe (dpy)]^{3+/2+}.

Examples of the amino acid residue having a positive charge include a lysine residue, a histidine residue, and an arginine residue and these may be used alone or as a combination of two or more types.

Among these, the lysine residue is particularly preferred. In general, when an enzyme is immobilized on an electrode, a method of immobilizing the enzyme using poly-L-lysine is carried out; however, when a codon encoding the lysine residue is added to or inserted into a base sequence encoding the enzyme, there is an advantage that the enzyme can be immobilized on the electrode without using poly-L-lysine and thus the cost for the poly-L-lysine material can be reduced.

Examples of the amino acid residue having a negative charge include an aspartic acid residue and a glutamic acid residue and these may be used alone or as a combination of two or more types.

A specific method for increasing the affinity using hydrophilic or hydrophobic interactions involves adding or inserting, to or into a base sequence encoding the enzyme, a codon that encodes a hydrophilic amino acid residue when a hydrophilic reaction substrate or electron transfer mediator is used and a codon that encodes a hydrophobic amino acid residue when a hydrophobic reaction substrate or electron transfer mediator is used so as to increase the affinity and/or reaction rate between the enzyme expressed and the reaction substrate or electron transfer mediator.

The electron transfer mediator that can be used in utilizing the hydrophilic or hydrophobic interactions is not particularly limited. Examples of the hydrophilic electron transfer mediator include those which have a Log P value, i.e., a general parameter of hydrophilicity/hydrophobicity, of less than 0, such cyano metal complexes, e.g., hexacyanoferric ions and octacyanotungstate ions, quinone, e.g., Q0, and pigments, e.g., 2,2'-azinobis(3-ethylbenzoline-6-sulfonate). Examples of the hydrophobic electron transfer mediator include those which have an Log P value greater than 0, such as VK1, VK3, benzoquinone, and anthraquinone.

Examples of the hydrophilic amino acid residues include an alanine residue, a valine residue, a leucine residue, an isoleucine residue, a methionine residue, a tryptophan residue, a phenylalanine residue, and a proline residue and these can be used alone or as a combination of two or more types.

Examples of the hydrophobic amino acid residues include a glycine residue, a serine residue, a threonine residue, a cysteine residue, a tyrosine residue, an asparagine residue, a glutamine residue, a lysine residue, a histidine residue, an arginine residue, an aspartic acid residue, and a glutamic acid residue and these may be used alone or as a combination of two or more.

The type of the enzyme that can be immobilized on the enzyme electrode according to the present invention is not particularly limited and any known enzyme can be freely selected. For example, when the enzyme electrode according to the present invention is used as a positive electrode, an enzyme that has an oxidase activity and oxygen serving as a reaction substrate, such as laccase, bilirubin oxidase (BOD), or ascorbate oxidase can be immobilized.

The enzyme that can be immobilized when the enzyme electrode according to the present invention is used as a negative electrode is also not particularly limited; however, for example, when a substrate containing a sugar as a reaction substrate is used, an oxidase that degrades sugars by oxidation is preferably immobilized. Examples of the oxidase include glucose dehydrogenase, gluconate-5-dehydrogenase, gluconate-2-dehydrogenase, alcohol dehydrogenase, aldehyde reductase, aldehyde dehydrogenase, lactate dehydrogenase, hydroxypyruvate reductase, glycerate dehydrogenase, formate dehydrogenase, fructose dehydrogenase, and galactose dehydrogenase.

Moreover, when the enzyme electrode according to the present invention is used as a negative electrode, an oxidized coenzyme and a coenzyme oxidase may be immobilized in addition to the oxidase described above. Examples of the oxidized coenzyme include nicotinamide adenine dinucleotide (NAD⁺), nicotinamide adenine dinucleotide phosphate (NADP⁺), flavin adenine dinucleotide (FAD⁺), and pyrrollo-quionoline quinone (PQQ2⁺). An example of the coenzyme oxidase is diaphorase.

Furthermore, an electron transfer mediator may be immobilized on the enzyme electrode according to the present invention in order to make smooth the transfer of electrons generated by the oxidation-reduction reactions to the electrode. The type of the electron transfer mediator that can be immobilized on the enzyme electrode according to the present invention is not particularly limited and any known electron transfer mediator can be freely selected. For example, when the enzyme electrode according to the present invention is used as a positive electrode, ABTS (2,2'-azinobis(3-ethylbenzoline-6-sulfonate)), K₃[Fe(CN)₆], or the like can be immobilized as the electron transfer mediator.

Furthermore, for example, when the enzyme electrode according to the present invention is used as a negative electrode, 2-amino-3-carboxy-1,4-naphthoquinone (ACNQ), vitamin K3, 2-amino-1,4-naphthoquinone (ANQ), 2-amino-3-methyl-1,4-naphthoquinone (AMNQ), 2,3-diamino-1,4-naphthoquinone, a metal complex of osmium (Os), ruthenium (Ru), iron (Fe), cobalt (Co), or the like, a viologen compound such as benzyl viologen, a compound having a quinone backbone, a compound having a nicotinamide structure, a compound having a riboflavin structure, or a compound having a nucleotide-phosphate structure can be immobilized.

Note that any known material can be used as the material used for the enzyme electrode according to the present invention without particular limitations as long as the material can be electrically connected to the outside. Examples thereof include metals such as Pt, Ag, Au, Ru, Rh, Os, Nb, Mo, In, Ir, Zn, Mn, Fe, Co, Ti, V, Cr, Pd, Re, Ta, W, Zr, Ge, and Hf, alloys such as alumel, brass, duralumin, bronze, nickeline, platinum rhodium, hyperco, permalloy, permendur, German silver, and phosphor bronze, conductive polymers such as polyacetylenes, carbon materials such as graphite and carbon black, borides such asHfB2, NbB, CrB₂, and B₄C, nitrides such as TiN and ZrN, silicides such as VSi₂, NbSi₂, MoSi₂, and TaSi₂, and mixtures of these.

### <Fuel cell>

A fuel cell according to the present invention is a fuel cell in which oxidation-reduction reactions proceed on an electrode with an enzyme acting as a catalyst and is a fuel cell in which an enzyme modified to increase the affinity and/or reaction rate with a reaction substrate or an electron transfer mediator is immobilized on at least one of a positive electrode and a negative electrode.

Modification of the enzyme is done by adding or inserting at least one codon encoding a particular amino acid residue to or into a base sequence encoding the enzyme. The details of the electrode of the fuel cell according to the present invention are the same as the details of the enzyme electrode discussed above; thus, the description therefor is omitted here.

The structure, functions, etc., of the fuel cell according to the present invention other than the electrode are not particularly limited and can be freely designed as long as the enzyme electrode according to the present invention is used.

Since the oxidation-reduction reactions proceed highly efficiently on the electrode of the fuel cell according to the present invention, the output of the obtained electrical energy can be increased. Moreover, since the stability of the electrode itself is high, a fuel cell having good durability can be provided.

### <Electronic apparatus>

The fuel cell according to the present invention can produce large output current and voltage and, in addition, has high durability; thus, it can be suitably used in all types of known electronic apparatuses.

The structure, functions, etc., of the electronic apparatus are not particularly limited as long as the fuel cell according to the present invention can be used, and all apparatuses that operate electrically are included. Examples thereof include electronic apparatuses such as cellular phones, mobile appliances, robots, personal computers, gaming machines, vehicle-mounted apparatuses, home electric appliances, and industrial products; moving products such as automobiles, bicycles, airplanes, rockets, and space crafts; medical apparatuses such as analyzers, pacemaker power generators, and in-vivo devices such as biosensors; and power generation systems and cogeneration systems such as a system that generates electric energy by decomposition of garbage.

### EXAMPLE 1

In Example 1, a gene was prepared in which a codon that encodes a particular amino acid residue was incorporated into a base sequence encoding an enzyme to modify the enzyme that can be immobilized on an enzyme electrode according to the present invention.

In particular, a gene in which lysine residues were incorporated into termini of bilirubin oxidase (hereinafter referred to as "BOD") was prepared by using BOD as an example of the enzyme that can be immobilized on the enzyme electrode according to the present invention and lysine residues as the amino acid residue.

First, pMETαB vectors Lys4, Lys6, Lys8, and Lys10 in which four, six, eight, and ten lysine residues were respectively incorporated were prepared as the vectors of BOD genes as indicated in Table 1.

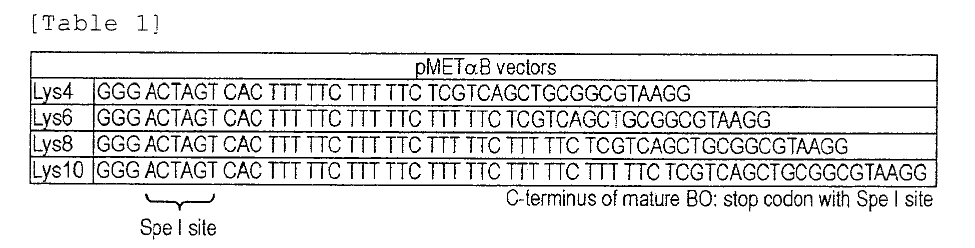

Genes (hereinafter referred to as "Lys4-BOD gene", "Lys6-BOD gene", "Lys8-BOD gene", and "Lys10-BOD gene") containing BOD genes were prepared by a PCR technique using the pMETαB vectors Lys4, Lys6, Lys8, and Lys10 prepared as in above. Next, these genes (Lys4-BOD gene, Lys6-BOD gene, Lys8-BOD gene, and Lys10-BOD gene) were incorporated into plasmids by ligation. The plasmids were transformed into Escherichia coli and developed with limiting media.

As a result, colonies of control (no plasmid), Lys4, Lys6, Lys8, and Lys10 were respectively obtained. Colonies were picked respectively, cut at Spe I and EcoRI, and subjected to gel electrophoresis to confirm whether target vectors (pMETαB vectors Lys4, Lys6, Lys8, and Lys10) had been transformed.

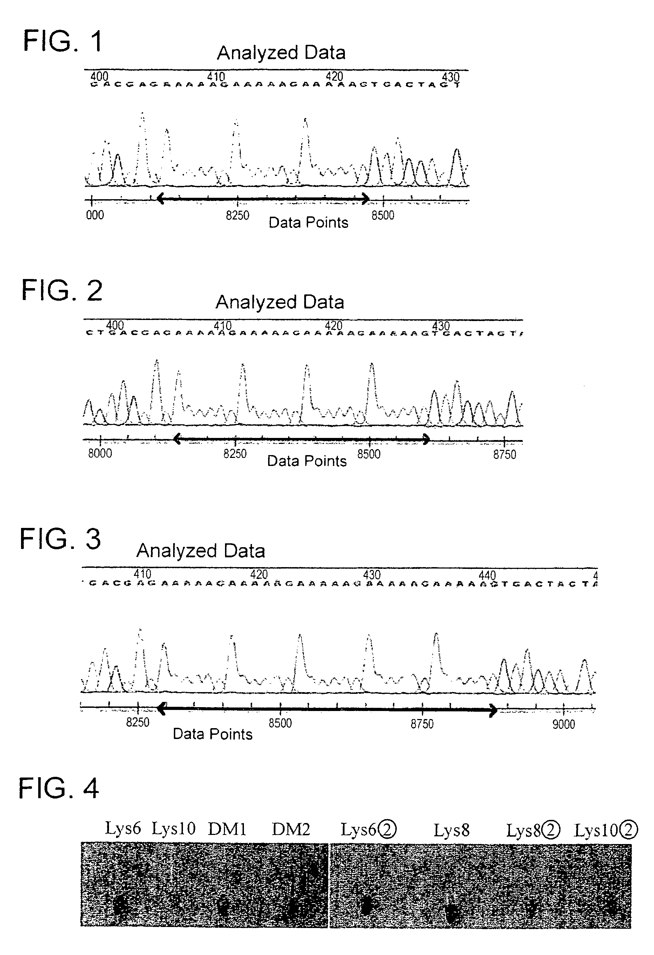

Next, each of the Lys6, Lys8, and Lys10 genes were subjected to DNA sequencing to confirm the target Lys sequence. The results of DNA sequencing are respectively shown in Figs. 1 to 3 (Lys6: Fig. 1, Lys8: Fig. 2, Lys10: Fig. 3).

### EXAMPLE 2

In Example 2, the plasmids prepared in Example 1 were transformed into yeast Pichia Methanolica to investigate presence or absence of BOD activity.

Using competent cells of pMAD11, the Lys4 gene, the Lys6 gene, the Lys8 gene, and the Lys10 gene cut out with limiting enzyme Asc1 were respectively transformed into yeast Pichia Methanolica. They were developed in MD media and two colonies were obtained from each.

The colonies were cultured with BMDY and transferred to BMMY to induce expression of BOD. To this solution, ABTS (2,2'-azinobis(3-ethylbenzoline-6-sulfonate) was added and the course of changing the color to green was observed to conduct expression check of whether there was BOD activity or not. The results are shown in Fig. 4. Note that the characters DM1 and DM2 in Fig. 4 indicate BOD into which the lysine residues were not incorporated.

As shown in Fig. 5, the BOD activity was confirmed in all enzymes. Accordingly, in Example 2, it was confirmed that the modified proteins expressed from the genes in which lysine residues were incorporated into termini of the base sequence encoding BOD maintained the BOD activity.

### Industrial Applicability

An enzyme electrode according to the present invention allows oxidation-reduction reactions on the electrode to proceed highly efficiently and thus the resulting output of the electrical energy can be increased. Accordingly, it can be used in all types of fuel cells, biosensors, and electronic apparatuses.

Moreover, the enzyme electrode according to the present invention can provide highly durable fuel cells, biosensors, and electronic apparatuses if used in the fuel cells, biosensors, and electronic apparatuses since the stability of the electrode itself is high.

Furthermore, the enzyme electrode according to the present invention can reduce the material needed for immobilizing the enzyme on the electrode; thus, it can contribute to reduction of manufacturing cost of the enzyme electrode itself and fuel cells, biosensors, electronic apparatuses, etc.

## Claims

1. An enzyme electrode wherein oxidation-reduction reactions proceed with an enzyme acting as a catalyst, and
the enzyme is modified to increase affinity and/or reaction rate with a reaction substrate or an electron transfer mediator by adding or inserting at least one codon encoding a particular amino acid residue to or into a base sequence encoding the enzyme, and is immobilized.

2. The enzyme electrode according to Claim 1, wherein the affinity and/or reaction rate of the enzyme with the reaction substrate or the electron transfer mediator is increased by electrostatic interactions.

3. The enzyme electrode according to Claim 1 or 2, wherein the amino acid residue has a charge opposite to that of the reaction substrate or the electron transfer mediator.

4. The enzyme electrode according to Claim 3, wherein the electron transfer mediator is a mediator whose oxidized form or reduced form is an anion and the amino acid residue is one amino acid selected from a lysine residue, a histidine residue, and an arginine residue.

5. The enzyme electrode according to Claim 1, wherein the affinity of the enzyme with the reaction substrate or the electron transfer mediator is increased by hydrophilic or hydrophobic interactions.

6. The enzyme electrode according to Claim 5, wherein the reaction substrate or the electron transfer mediator is a hydrophilic substance and the amino acid is a hydrophilic amino acid.

7. The enzyme electrode according to Claim 5, wherein the reaction substrate or the electron transfer mediator is a hydrophobic substance and the amino acid is a hydrophobic amino acid.

8. A fuel cell wherein oxidation-reduction reactions proceed on an electrode with an enzyme acting as a catalyst, and
the enzyme is modified to increase affinity and/or reaction rate with a reaction substrate or an electron transfer mediator by adding or inserting at least one codon encoding a particular amino acid residue to or into a base sequence encoding the enzyme, and is immobilized on at least one of a positive electrode and a negative electrode.
